(19) Europäisches Patentamt
European Patent Office
Office européen des brevets

(11) Veröffentlichungsnummer : **0 464 473 B1**

(12) # EUROPÄISCHE PATENTSCHRIFT

(45) Veröffentlichungstag der Patentschrift : **03.08.94 Patentblatt 94/31**

(51) Int. Cl.[5] : **C07C 235/76,** C10M 173/02, C23F 11/14

(21) Anmeldenummer : **91110057.6**

(22) Anmeldetag : **19.06.91**

(54) **Salze von Alkenylbernsteinsäurehalbamiden und deren Verwendung als Korrosionsschutzmittel und Emulgatoren für Metallbearbeitungsöle.**

(30) Priorität : **23.06.90 DE 4020061**

(43) Veröffentlichungstag der Anmeldung : **08.01.92 Patentblatt 92/02**

(45) Bekanntmachung des Hinweises auf die Patenterteilung : **03.08.94 Patentblatt 94/31**

(84) Benannte Vertragsstaaten : **AT BE CH DE ES FR GB IT LI NL SE**

(56) Entgegenhaltungen :
**EP-A- 0 127 132**
**EP-A- 0 191 952**

(56) Entgegenhaltungen :
**EP-A- 0 216 280**
**GB-A- 1 090 419**
**US-A- 2 982 634**
**US-A- 4 579 922**

(73) Patentinhaber : **HOECHST AKTIENGESELLSCHAFT**
**D-65926 Frankfurt (DE)**

(72) Erfinder : **Ritschel, Werner, Dr. Deceased (DE)**
Erfinder : **Lorke, Horst**
**Wachenheimer Strasse 14**
**W-6237 Liederbach (DE)**
Erfinder : **Kremer, Gernot,Dr.**
**Frankfurter Strasse 200**
**W-6233 Kelkheim/Taunus (DE)**

**EP 0 464 473 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Korrosionsschutzmittel und Emulgatoren in emulgierbaren Metollbearbeitungsölen, die zwischen 20 - 50 % dieser Produkte und 80 - 20 % naphthenbasische, paraffinbasische oder gemischtbasische Mineralöle enthalten, sollten folgende Forderungen erfüllen:
Ausgeprägte Korrosionsschutzeigenschaften gegenüber Eisenmetallen,
Emulgiervermögen gegenüber Mineralölen, eventuell auch in Kombination mit ausgewählten nichtionogenen Verbindungen,
geringe Schaumneigung bzw. schneller Schaumzerfall,
Verhinderung des Wachstums von Mikroorganismen.

Diese Forderungen werden von den in EP 0127132 aufgeführten Verbindungen nur teilweise erfüllt. Aus diesem Dokument sind bereits Alkenylbernsteinsäurehalbamide der Formel

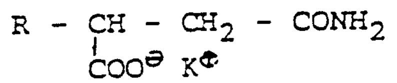

wobei R $C_6$-$C_{12}$-Alkenyl bedeutet, und deren Verwendung als Korrosionsschutzmittel bekannt.

Diese Verbindungen zeigen aber ein nicht ausreichendes Emulgiervermögen, was zu ungenügenden Standzeiten der Gebrauchsemulsionen führt. Außerdem neigen Emulsionen mit diesen Verbindungen zu Schaumbildung, was besonders bei Arbeitsprozessen wie dem Schleifen, wobei die Emulsionen hohen mechanischen Beeinflussungen ausgesetzt sind, von Nachteil ist.

Aus US-A-2 982 634 ist das $C_{23}$-Alkenylbernsteinsäurehalbamid und dessen Verwendung als Zusatzmittel für Motorverbrennungsstoffe bekannt.

EP-A-0 191 952 beschreibt Amidamin-Salze von $C_6$-$C_{22}$-Alkenylbernsteinsäurehalbamiden und deren Verwendung als Korrosionsinhibitoren für Wasser-in-Öl-Emulsionen, wie sie beim Erdöl vorliegen.

US-A-4 549 882 betrifft den Einsatz von mindestens einer $C_8$-$C_{30}$-Alkenylbernsteinsäure in Mischung mit einem N-(2-Hydroxyalkyl)-monoalkanolamin oder N-(2-Hydroxyalkyl)-dialkanolamin als Korrosionsinhibitor für alkoholenthaltende Motorverbrennungsstoffe.

Die beschriebenen Nachteile lassen sich überwinden, indem man die im folgenden beschriebenen, erfindungsgemäßen längerkettigen Alkenylbernsteinsäurehalbamide einsetzt. Die Opaleszenz der Emulsionen, die mit diesen längerkettigen Alkenylbernsteinsäurehalbamiden erzielt wird, zeigt gegenüber den kürzerkettigen eine bessere Feinverteilung der Emulsionen und somit lange Standzeiten, d.h. eine lange Gebrauchsmöglichkeit der Emulsion an. Auch die Forderung nach geringer Schaumneigung bzw. schnellem Schaumzerfall erfüllen die erfindungsgemäßen längerkettigen Alkenylbernsteinsäurehalbamide, nicht dagegen die kürzerkettigen Verbindungen.

Gegenstand der Erfindung sind deshalb Alkenylbernsteinsäurehalbamide der Formeln

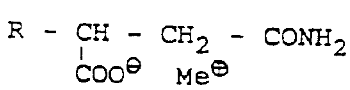

oder

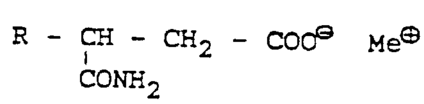

worin R $C_{15}$-$C_{30}$-, bevorzugt $C_{15}$-$C_{21}$-Alkenyl ist, wobei R geradkettig oder verzweigt sein kann, und Me+ ein Alkalimetallion ($Na^+$,$K^+$), ein Proton oder ein Ammoniumion der Formel $^+NHR_1R_2R_3$ bedeutet und $R_1$, $R_2$ und $R_3$ gleich oder verschieden sind und Wasserstoff, 2-Hydroxyethyl oder 2-Hydroxypropyl bedeuten, ausgenommen Alkenylbernsteinsäurehalbamide, worin R $C_{18}$-Alkenyl und $Me^\oplus$ eine Ammoniumion der Formel $NH_4^\oplus$ oder $C_{23}$-Alkenyl und $Me^\oplus$ ein Proton bedeutet.

Weiterhin betrifft die Erfindung die Verwendung von Alkenylbernsteinsäurehalbamiden als korrosionsschutzmittel.

Die Herstellung der als Ausgangsmaterialien verwendeten Alkenylbernsteinsäureanhydride aus Olefin und Maleinsäureanhydrid ist bekannt. Die Alkenylbernsteinsäurehalbamide werden erhalten durch Umsetzung von 1 Mol eines Alkenylbernsteinsäureanhydrids mit mindestens 2 Mol Ammoniak, wobei man das Alkenylbernsteinsäurehalbamid in Form des Ammoniumsalzes erhält. Verwendet man wäßrige Ammoniaklösung, fällt das Alkenylbernsteinsäurehalbamid-Ammoniumsalz in wäßriger Lösung an. Durch Verdrängen des Ammoniaks mit

Hilfe anderer Basen lassen sich andere Salze der Halbamide darstellen. Besonders bevorzugt zur Verwendung als Korrosionsschutzmittel sind die Alkanolaminsalze wie Triethanolamin- oder Triisopropanolaminsalze, die durch Umsetzung der zunächst anfallenden Ammoniumsalze mit einer wäßrigen Lösung des Alkanolamins bei erhöhter Temperatur hergestellt werden.

Der freigesetzte Ammoniak kann vollständig entfernt werden, indem man die wäßrige Lösung des Alkanolammoniumsalzes erhitzt und einen kräftigen Stickstoffstrom durch die Lösung leitet. Durch zusätzliches Abdestillieren einer bestimmten Wassermenge kann die Entfernung des Ammoniaks unterstützt werden, dabei kann gleichzeitig eine bestimmte Konzentration der Wirksubstanz eingestellt werden.

Die oben beschriebenen Alkenylbernsteinsäurehalbamide sind klar wasserlöslich bzw. ergeben mit Mineralöl leicht emulgierbare Produkte. Diese Produkte können als Korrosionsschutzmittel in wäßrigen Kühlschmiermitteln, insbesondere Bohr-, Schneid- und Walzflüssigkeiten, eingesetzt werden. Zur Bereitung dieser wäßrigen Kühlschmiermittel werden die Reaktionsprodukte in die erforderliche Menge Wasser eingerührt oder mit Mineralöl gemischt und die erhaltenen Konzentrate in Wasser emulgiert. Die Anwendungskonzentration in den Bohr-, Schneid- und Walzflüssigkeiten beträgt im allgemeinen etwa 0,1 bis 10 Gew.-%, vorzugsweise 2-10 Gew.-%. Die genannten Konzentrationen beziehen sich auf die Anwendung der Produkte für sich, als auch auf die erwähnten Kombinationen mit Mineralöl.

Diese Emulsionskonzentrate enthalten zusätzliche Hilfsstoffe zur Optimierung des Emulgierverhaltens und des Korrosionsschutzes. Dafür werden beispielsweise verwendet Oxethylate der allgemeinen Formel

$$R_4 - O - (CH_2 - CH_2 - O)_nH$$

wobei $R_4$ = $C_{10}$-$C_{20}$-Alkyl, Alkenyl oder Alkylphenyl und n Zahlen von 2 bis 10 bedeuten und/oder Fettsäurealkanolamide der allgemeinen Formel

$$R_5 - CO - NR_6R_7$$

wobei $R_6$ = $C_{10}$-$C_{20}$-Alkyl oder $C_{10}$-$C_{20}$-Alkenyl und $R_6$ und $R_7$ gleich oder verschieden sind und Wasserstoff, 2-Hydroxyethyl oder 2-Hydroxypropyl bedeuten. Diese Produkte können in den Emulsionskonzentraten in Mengen von jeweils ca. 20 bis 40 % zugeben sein.

Beispiele

Beispiel 1

Triethanolamin/Na-Salz des Pentapropenylbernsteinsäurehalbamids

In einem 4-Hals-Kolben mit Destillationsaufsatz, Tropftrichter, Thermometer und Rührer werden 300 g E-Wasser und 300 g (4,4 Mol) 25 %ige wäßrige Ammoniak-Lösung vorgelegt und auf 0 °C abgekühlt. Dann tropft man innerhalb 30 Min. 696 g (2,0 Mol) Pentapropenylbernsteinsäureanhydrid zu, wobei die Temperatur durch Kühlen bei 10-20 °C gehalten wird. Nach beendetem Zutropfen rührt man noch 3 h bei 30 °C nach.
Zu dieser Ammoniumsalz-Lösung gibt man 298 g (2,0 Mol) Triethanolamin, rührt 5 Min., dann 96 g (1,2 Mol) NaOH (50 %ig) zu. Unter Rühren wird langsam (ca. 2 h) bis auf 110 °C erhitzt, wobei Ammoniak entweicht und Wasser sowie Olefin abdestilliert werden. Man destilliert noch 2 h bei 110 °C, dabei gehen ca. 500 g ammoniakalisches Wasser sowie ca. 50 g Olefin über. Man erhält etwa 1090 g dunkle Flüssigkeit mit einem Wirkstoffgehalt von ca. 80 %. Das in diesem Beispiel verwendete rohe Pentapropenylbernsteinsäureanhydrid enthält im wesentlichen folgende Bestandteile:

| | |
|---|---|
| Pentapropenylbernsteinsäureanhydrid | ca. 57 % |
| Hexapropenylbernsteinsäureanhydrid | ca. 10 % |
| Heptapropenylbernsteinsäureanhydrid | ca. 3 % |
| Pentapropylen | ca. 22 % |

Beispiel 2

370 g des unter Beispiel 1 hergestellten Produktes,
310 g Tallölfettsäurediethanolamid,
160 g Oleylalkoholpolyglykolether (2 Mol Ethylenoxid) sowie

160 g Nonylphenolpolyglykolether (2 Mol Ethylenoxid) werden bei Raumtemperatur gemischt und gerührt, bis eine klare Lösung entstanden ist.

Vergleichssubstanz A

Triethanolamin/Na-Salz des Tripropenylbernsteinsäurehalbamids

In einem 4-Hals-Kolben mit Destillationsaufsatz, Tropftrichter, Thermometer und Rührer werden 150 g Wasser und 150 g (2,2 Mol) 25 %ige wäßrige Ammoniak-Lösung vorgelegt und auf 5 °C gekühlt. Dann tropft man innerhalb 15 Min. 224 g (1,0 Mol) Tripropenylbernsteinsäureanhydrid zu, wobei die Temperatur durch Kühlen bei 10-20 °C gehalten wird. Nach beendetem Zutropfen rührt man noch 3 h bei 30 °C nach.
Nun gibt man 149 g (1,0 Mol) Triethanolamin zu, rührt 5 Min und gibt dann 48 g (0,6 Mol) NaOH (50 %ig) zu. Innerhalb 1-2 h wird auf 110 °C hochgeheizt, wobei durch die Lösung Stickstoff geleitet wird. Innerhalb 2 h wird bei 110 °C 299 g ammoniakalisches Wasser abdestilliert. Mit 83 g Wasser wird der Feststoffgehalt auf 79 % eingestellt. Man erhält 469 g Produkt mit einem Wassergehalt von ca. 18 %.

Vergleichssubstanz B

Triethanolamin/Na-Salz des Tetrapropenylbernsteinsäurehalbamids

In einem 4-Hals-Kolben mit Destillationsaufsatz, Tropftrichter, Thermometer und Rührer werden 150 g Wasser und 150 g (2,2 Mol) 25 %ige wäßrige Ammoniak-Lösung vorgelegt und auf 0-5 °C gekühlt. Dann tropft man innerhalb 15 Min. 266 g (1 Mol) Tetrapropenylbernsteinsäureanhydrid zu, wobei die Temperatur durch Kühlen bei 10-20 °C gehalten wird. Nach beendetem Zutropfen rührt man noch 3 h bei 30 °C nach.
Nun gibt man 149 g (1 Mol) Triethanolamin zu, rührt 5 Min. und gibt dann 48 g (0,6 Mol) NaOH (50 %ig) zu. Innerhalb 1-2 h wird auf 110 °C hochgeheizt, wobei durch die Lösung Stickstoff geleitet wird. Innerhalb 2 h wird bei 110 °C 270 g ammoniakalisches Wasser abdestilliert. Mit 48,8 g Wasser wird der Feststoffgehalt auf 80,8 % eingestellt. Auswaage: 494 g

Vergleichssubstanz C

370 g der Vergleichssubstanz A,
310 g Tallölfettsäurediethanolamid,
160 g Oleylalkoholpolyglykolether (2 Mol Ethylenoxid) sowie 160 g Nonylphenolpolyglykolether (2 Mol Ethylenoxid) werden bei Raumtemperatur gemischt und gerührt, bis eine klare Lösung entstanden ist.

Vergleichssubstanz D

370 g der Vergleichssubstanz B,
310 g Tallölfettsäurediethanolamid,
160 g Oleylalkoholpolyglykolether (2 Mol Ethylenoxid) sowie 160 g Nonylphenolpolyglykolether (2 Mol Ethylenoxid) werden bei Raumtemperatur gemischt und gerührt, bis eine klare Lösung entstanden ist.

| Beispiel<br>Vergl. Substanz | 1<br>- | 2<br>- | -<br>A | -<br>B | -<br>C | -<br>D |
|---|---|---|---|---|---|---|
| Aussehen/20°C | braune, klare Flüssigkeit | | | | | |
| pH-Wert | | | | | | |
| 1 % in dest. Wasser | 8.7 | 9.1 | 8.3 | 8.4 | 8.8 | 9.0 |
| Löslichkeit | | | | | | |
| 3 %ige Lösung in $H_2O$ | | | | | | |
| 0° dH sofort | klar | - | transp. | transp. | - | - |
| 0° dH n. 24 Std. | klar | - | transp. | transp. | - | - |
| 20° dH sofort (Natur) | transp. | - | trüb | transp. | - | - |
| 20° dH n. 24 Std. (Natur) | transp. | - | stark trüb | trüb | - | - |
| 20° dH sofort<br>Synthese/DIN 51360/1 | transp. | -<br>- | trüb<br>(Flockung) | trüb<br>(Flockung) | - | - |
| 20° dH nach 24 Std.<br>Synthese/DIN 51360/1 | transp. | -<br>- | trüb<br>(Flockung) | trüb<br>(Flockung) | - | - |
| Schaumverhalten* | | | | | | |
| 3 %ige Lösung<br>*50 ml werden in einem 250 ml verschlossenen Meßzylinder 1 Min. kräftig geschüttelt | | | | | | |
| dest. $H_2O$ sofort | Schaum | - | stark Schaum | stark Schaum | - | - |
| nach 5 Min. | zerfallen | - | Schaum | Schaum | - | - |
| 20° dH Natur sofort | wenig Schaum | - | Schaum | Schaum | - | - |
| nach 5 Min. | zerfallen | - | Schaum | Schaum | - | - |
| 20° dH Synthese sofort | wenig Schaum | - | Schaum | Schaum | - | - |
| nach 5 Min. | zerfallen | - | Schaum | Schaum | - | - |

| Beispiel / Vergl. Substanz | 1 / - | 2 / - | - / A | - / B | - / C | - / D |
|---|---|---|---|---|---|---|
| **Korrosionsschutz** | | | | | | |
| DIN 51360/1 | | | | | | |
| dest. $H_2O$ 1 % | kein Rost | - | kein Rost | Spur Rost | - | - |
| 20° dH Natur 2,5 % | kein Rost | - | Spur Rost | Spur Rost | - | - |
| 20° dH Synthese 3 % | kein Rost | - | Spur Rost | Spur Rost | - | - |
| DIN 51360/2 | | | | | | |
| dest. $H_2O$ 1,5 % | kein Rost | - | kein Rost | kein Rost | - | - |
| 20° dH Natur 2,5 % | kein Rost | - | kein Rost | kein Rost | - | - |
| 20° dH Synthese 3,0 % | kein Rost | - | Spur Rost | Spur Rost | - | - |
| **Emulgierverhalten Korrosionschutz Schaumvermögen** | | | | | | |
| 80 % Mineralöl) Konzentrat 20 % Emulgator) daraus 5 %ige Emulsion | | | | | | |
| **a) Emulgierverhalten** | | | | | | |
| dest. $H_2O$ sofort | - | milchig-opal | - | - | milchig | milchig |
| nach 24 h | | unverändert | | | Rahm | Rahm |
| 20° dH Natur sofort | - | milchig-opal | - | - | milchig | milchig |
| nach 24 h | | unverändert | | | Rahm | starker Rahm |
| 20° dH Synthese sofort | - | milchig-opal | - | - | milchig | milchig |
| nach 24 h | | unverändert | | | Rahm | Rahm/Oel |
| **b) Korrosionsschutz** | | | | | | |
| DIN 51360/1: 2 %ige Emulsion | | | | | | |
| dest. $H_2O$ | - | kein Rost | - | - | kein Rost | kein Rost |
| Synth. $H_2O$ 20° dH | | kein Rost | | | Rost | Rost |
| **c) Schaumverhalten** | | | | | | |
| 20° dH Natur sofort | - | Spur Schaum | - | - | Schaum | Schaum |
| nach 5 Min. | - | kein Schaum | - | - | Schaum | Schaum |

## Patentansprüche

### Patentansprüche für folgende Vertragsstaaten : AT, BE, CH, DE, FR, GB, IT, LI, NL, SE

**1.** Alkenylbernsteinsäurehalbamide der Formeln

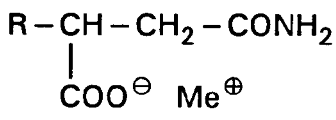

oder

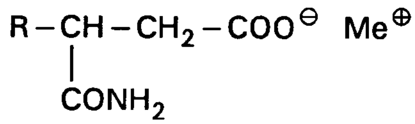

worin R $C_{15}$-$C_{30}$-Alkenyl ist, wobei R verzweigt oder geradkettig sein kann, und $Me^{\oplus}$ ein Alkalimetallion, ein Proton oder ein Ammoniumion der Formel $^{+}NHR_1R_2R_3$ bedeutet und $R_1$, $R_2$ und $R_3$ gleich oder verschieden sind und Wasserstoff, 2-Hydroxyethyl oder 2-Hydroxypropyl bedeuten, ausgenommen Alkenylbernsteinsäurehalbamide, worin R $C_{18}$-Alkenyl in Kombination mit $Me^{\oplus}$ ein Ammoniumion der Formel $NH_4^{\oplus}$ oder R $C_{23}$-Alkenyl in Kombination mit $Me^{\oplus}$ ein Proton bedeutet.

**2.** Alkenylbernsteinsäurehalbamide nach Anspruch 1, wobei R $C_{15}$-$C_{21}$-Alkenyl ist.

**3.** Verwendung von Alkenylbernsteinsäurehalbamiden der Formeln

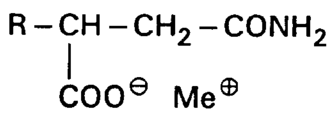

oder

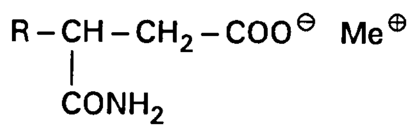

worin R $C_{15}$-$C_{30}$-Alkenyl ist, wobei R verzweigt oder geradkettig sein kann, und $Me^{\oplus}$ ein Alkalimetallion, ein Proton oder ein Ammoniumion der Formel $^{+}NHR_1R_2R_3$ bedeutet und $R_1$, $R_2$ und $R_3$ gleich oder verschieden sind und Wasserstoff, 2-Hydroxyethyl oder 2-Hydroxypropyl bedeuten, als Korrosionsschutzmittel in wasser- und/oder mineralölhaltigen Formulierungen.

**4.** Verwendung nach Anspruch 3, wobei R $C_{15}$-$C_{21}$-Alkenyl ist.

**5.** Verwendung der Alkenylbernsteinsäurehalbamide nach Anspruch 3 als Korrosionsschutzmittel in wasser- und/oder mineralölhaltigen Formulierungen zusammen mit Oxethylaten der allgemeinen Formel

$$R_4\text{-O-}(CH_2\text{-}CH_2\text{-O})_nH$$

wobei $R_4$ = $C_{10}$-$C_{20}$-Alkyl, Alkenyl oder Alkylphenyl und n Zahlen von 2 bis 10 bedeuten und/oder Fettsäurealkanolamiden der allgemeinen Formel

$$R_5\text{-CO-}NR_6R_7$$

wobei $R_6$ = $C_{10}$-$C_{20}$-Alkyl oder $C_{10}$-$C_{20}$-Alkenyl und $R_6$ und $R_7$ gleich oder verschieden sind und Wasserstoff, 2-Hydroxyethyl oder 2-Hydroxypropyl bedeuten.

**6.** Wasser- und/oder mineralölhaltige Korrosionsschutzmittel, enthaltend Alkenylbernsteinsäurehalbamide nach Anspruch 1.

### Patentansprüche für folgenden Vertragsstaat : ES

**1.** Verwendung von Alkenylbernsteinsäurehalbamide der Formeln

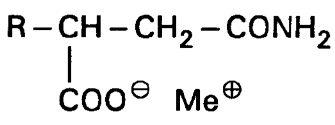

oder

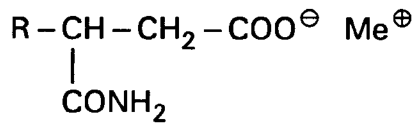

worin R $C_{15}$-$C_{30}$-Alkenyl ist, wobei R verzweigt oder geradkettig sein kann, und $Me^{\oplus}$ ein Alkalimetallion, ein Proton oder ein Ammoniumion der Formel $^{+}NHR_1R_2R_3$ bedeutet und $R_1$, $R_2$ und $R_3$ gleich oder verschieden sind und Wasserstoff, 2-Hydroxyethyl oder 2-Hydroxypropyl bedeuten, als Korrosionsschutzmittel in wasser- und/oder mineralölhaltigen Formulierungen.

2. Verwendung nach Anspruch 1, wobei R $C_{15}$-$C_{21}$-Alkenyl ist.

3. Verwendung von Alkenylbernsteinsäurehalbamide nach Anspruch 1 als Korrosionsschutzmittel in wasser- und/oder mineralölhaltigen Formulierungen zusammen mit Oxethylaten der allgemeinen Formel

$$R_4\text{-O-}(CH_2\text{-}CH_2\text{-O})_nH$$

wobei $R_4$ = $C_{10}$-$C_{20}$-Alkyl, Alkenyl oder Alkylphenyl und n Zahlen von 2 bis 10 bedeuten und/oder Fettsäurealkanolamiden der allgemeine Formel

$$R_5\text{-CO-}NR_6R_7$$

wobei $R_6$ = $C_{10}$-$C_{20}$-Alkyl oder $C_{10}$-$C_{20}$-Alkenyl und $R_6$ und $R_7$ gleich oder verschieden sind und Wasserstoff, 2-Hydroxyethyl oder 2-Hydroxypropyl bedeuten.

4. Wasser- und/oder mineralölhaltige Korrosionsschutzmittel, enthaltend Alkenylbernsteinsäurehalbamide nach Anspruch 1, ausgenommen Alkenylbernsteinsäurehalbamide worin R $C_{18}$-Alkenyl in Kombination mit $Me^{\oplus}$ ein Ammoniumion der Formel $NH_4^{\oplus}$, oder R $C_{23}$-Alkenyl in Kombination mit $Me^{\oplus}$ ein Proton bedeutet.

5. Wasser- und/oder mineralölhaltige Korrosionsschutzmittel, enthaltend 0,1 bis 10 Gew.-% eines Alkenylbernsteinsäurehalbamide nach Anspruch 1.

## Claims

**Claims for the following Contracting States : AT, BE, CH, DE, FR, GB, IT, LI, NL, SE**

1. An alkenylsuccinic monoamide of the formula

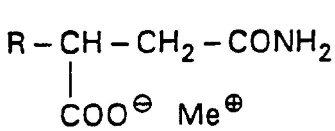

or

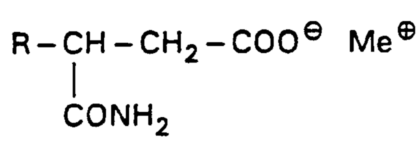

in which R is $C_{15}$-$C_{30}$-alkenyl where R can be branched or straight-chain, and $Ne^{\oplus}$ is an alkali metal ion, a proton or an ammonium ion of the formula $^{+}NHR_1R_2R_3$ and $R_1$, $R_2$ and $R_3$ are identical or different and are hydrogen, 2-hydroxyethyl or 2-hydroxypropyl, with the exception of an alkenylsuccinic monoamide in which R is $C_{18}$-alkenyl and $Me^{\oplus}$ is an ammonium ion or R is $C_{23}$-alkenyl and $Me^{\oplus}$ is a proton.

2. An alkenylsuccinic monoamide as claimed in claim 1 in which R is $C_{15}$-$C_{21}$-alkenyl

3. Use of alkenylsuccinic monoamide of the formula

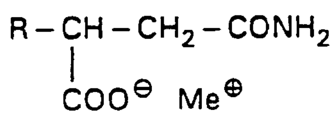
or
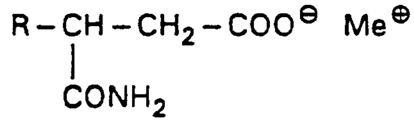

in which R is $C_{15}$-$C_{30}$-alkenyl where R can be branched or straight-chain, and $Me^{\oplus}$ is an alkali metal ion, a proton or an ammonium ion of the formula $^{+}NHR_1R_2R_3$ and $R_1$, $R_2$ and $R_3$ are identical or different and are hydrogen, 2-hydroxyethyl or 2-hydroxypropyl as anticorrosive agent in water- and/or mineral oil-containing formulations.

**4.** Use as claimed in claim 3, where R is $C_{15}$-$C_{21}$-alkenyl.

**5.** Use of an alkenylsuccinic monoamide as claimed in claim 3 as anticorrosive agent in water- and/or mineral oil-containing formulations together with ethoxylates of the formula

$$R_4\text{-O-}(CH_2\text{-}CH_2\text{-O})_nH$$

in which $R_4$ is $C_{10}$-$C_{20}$-alkyl, alkenyl or alkylphenyl and n represents numbers from 2 to 10 and/or fatty acid alkanolamides of the formula

$$R_5\text{-CO-}NR_6R_7$$

in which $R_5$ is $C_{10}$-$C_{20}$-alkyl or $C_{10}$-$C_{20}$-alkenyl and $R_6$ and $R_7$ are identical or different and are hydrogen, 2-hydroxyethyl or 2-hydroxypropyl.

**6.** A water- and/or mineral oil-containing anticorrosive agent, containing an alkenylsuccinic monoamide as claimed in claim 1.

**Claims for the following Contracting State : ES**

**1.** Use of an alkenylsuccinic monoamide of the formula

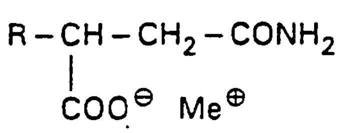
or
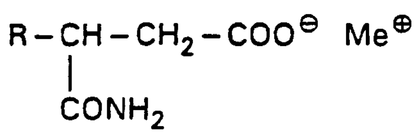

in which R is $C_{15}$-$C_{30}$-alkenyl where R can be branched or straight-chain, and $Me^{\oplus}$ is an alkali metal ion, a proton or an ammonium ion of the formula $^{+}NHR_1R_2R_3$ and $R_1$, $R_2$ and $R_3$ are identical or different and are hydrogen, 2-hydroxyethyl or 2-hydroxypropyl as anticorrosive agent in water- and/or mineral oil-containing formulations.

**2.** Use as claimed in claim 1, where R is $C_{15}$-$C_{21}$-alkenyl.

**3.** Use of an alkenylsuccinic monoamide as claimed in claim 1 as anticorrosive agent in water- and/or mineral oil-containing formulations together with ethoxylates of the formula

$$R_4\text{-O-}(CH_2\text{-}CH_2\text{-O})_nH$$

in which $R_4$ is $C_{10}$-$C_{20}$-alkyl, alkenyl or alkylphenyl and n represents numbers from 2 to 10 and/or fatty acid alkanolamides of the formula

$$R_5\text{-CO-}NR_6R_7$$

in which $R_5$ is $C_{10}$-$C_{20}$-alkyl or $C_{10}$-$C_{20}$-alkenyl and $R_6$ and $R_7$ are identical or different and are hydrogen, 2-hydroxyethyl or 2-hydroxypropyl.

**4.** A water- and/or mineral oil-containing anticorrosive agent, containing an alkenylsuccinic monoamide as claimed in claim 1, with the exception of an alkenylsuccinic monoamide in which R is $C_{18}$-alkenyl and $Me^{\oplus}$ is an ammonium ion or R is $C_{23}$-alkenyl and $Me^{\oplus}$ is a proton.

5. A water- and/or mineral oil-containing anticorrosive agent, containing 0.1 to 10% by weight of an alkenylsuccinic monoamide as in claim 1.

## Revendications

### Revendications pour les Etats contractants suivants : AT, BE, CH, DE, FR, GB, IT, LI, NL, S

1. Demi-amides d'acides alcénylsucciniques de formules

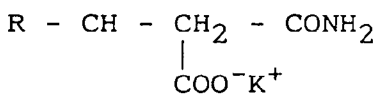

ou

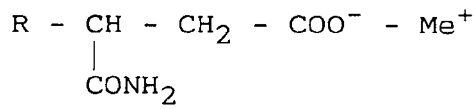

où R est un radical alcényle en $C_{15}$-$C_{30}$, R pouvant avoir une chaîne droite ou ramifiée, et $Me^+$ est un ion d'un métal alcalin, un proton ou un ion ammonium de formule $^+NHR_1R_2R_3$, et $R_1$, $R_2$ et $R_3$ sont identiques ou différents et représentent chacun l'hydrogène ou le radical 2-hydroxyéthyle ou 2-hydroxypropyle, à l'exception des demi-amides d'acides alcénylsucciniques dans lesquels R est un radical alcényle en $C_{18}$ et $Me^+$ est un ion ammonium de formule $NH_4^+$, ou encore R est un radical alcényle en $C_{23}$ et $Me^+$ est un proton.

2. Demi-amides d'acides alcénylsucciniques selon la revendication 1, dans lesquels R est un radical alcényle en $C_{15}$-$C_{21}$.

3. Utilisation des demi-amides d'acides alcénylsucciniques de formules

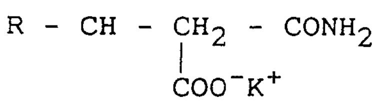

ou

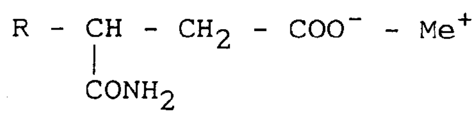

où R est un radical alcényle en $C_{15}$-$C_{30}$, R pouvant avoir une chaîne droite ou ramifiée, et $Me^+$ est un ion d'un métal alcalin, un proton ou un ion ammonium de formule $^+NHR_1R_2R_3$, et $R_1$, $R_2$ et $R_3$ sont identiques ou différents et représentent chacun l'hydrogène ou le radical 2-hydroxyéthyle ou 2-hydroxypropyle, comme agent anti-corrosion dans des formulations contenant de l'eau et/ou une huile minérale.

4. Utilisation selon la revendication 3, dans laquelle R est un radical alcényle en $C_{15}$-$C_{21}$.

5. Utilisation des demi-amides d'acides alcénylsucciniques selon la revendication 3 en tant qu'agent anti-corrosion dans des formulations contenant de l'eau et/ou une huile minérale, avec des produits d'éthoxylation de formule générale

$$R_4 - O - (CH_2 - CH_2 - O)_n H$$

où $R_4$ est un radical alkyle, alcényle ou alkylphényle en $C_{10}$-$C_{20}$ et n est un nombre de 2 à 10, et/ou des alcanolamides d'acides gras de formule générale

$$R_5 - O - NR_6R_7$$

où $R_5$ est un radical alkyle en $C_{10}$-$C_{20}$ ou alcényle en $C_{10}$-$C_{20}$, et $R_6$ et $R_7$ sont identiques ou différents et sont chacun un hydrogène ou le radical 2-hydroxyéthyle ou 2-hydroxypropyle.

6. Agent anti-corrosion contenant de l'eau et/ou une huile minérale, contenant des demi-amides d'acides alcénylsucciniques selon la revendication 1.

### Revendications pour l'Etat contractant suivant : ES

1. Utilisation des demi-amides d'acides alcénylsucciniques de formules

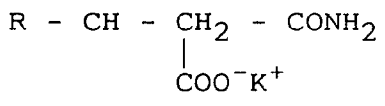

ou

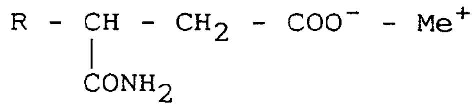

où R est un radical alcényle en $C_{15}$-$C_{30}$, R pouvant avoir une chaîne droite ou ramifiée, et $Me^+$ est un ion d'un métal alcalin, un proton ou un ion ammonium de formule $^+NHR_1R_2R_3$, et $R_1$, $R_2$ et $R_3$ sont identiques ou différents et représentent chacun l'hydrogène ou le radical 2-hydroxyéthyle ou 2-hydroxypropyle, en tant qu'agent anticorrosion dans des formulations contenant de l'eau et/ou une huile minérale.

**2.** Utilisation selon la revendication 1, dans laquelle R est un radical alcényle en $C_{15}$-$C_{21}$.

**3.** Utilisation des demi-amides d'acides alcénylsucciniques selon la revendication 1 en tant qu'agent anti-corrosion dans des formulations contenant de l'eau et/ou une huile minérale, avec des produits d'éthoxylation de formule générale

$$R_4 - O - (CH_2 - CH_2 - O)_n H$$

où $R_4$ est un radical alkyle, alcényle ou alkylphényle en $C_{10}$-$C_{20}$ et n est un nombre de 2 à 10, et/ou des alcanolamides d'acides gras de formule générale

$$R_5 - O - NR_6R_7$$

où $R_5$ est un radical alkyle en $C_{10}$-$C_{20}$ ou alcényle en $C_{10}$-$C_{20}$, et $R_6$ et $R_7$ sont identiques ou différents et sont chacun un hydrogène ou le radical 2-hydroxyéthyle ou 2-hydroxypropyle.

**4.** Agents anti-corrosion contenant de l'eau et/ou une huile minérale, contenant des demi-amides d'acides alcénylsucciniques selon la revendication 1, à l'exception des demi-amides d'acides alcénylsucciniques dans lesquels R est un radical alcényle en $C_{18}$ et $Me^+$ est un ion ammonium de formule $NH_4^+$, ou encore R est un radical alcényle en $C_{23}$ et $Me^+$ est un proton.

**5.** Agents anti-corrosion contenant de l'eau et/ou une huile minérale, contenant de 0,1 à 10 % en poids de l'un des demi-amides d'acides alcénylsucciniques selon la revendication 1.